# EUROPÄISCHE PATENTSCHRIFT

(11) **EP 1 697 365 B1**
(45) Veröffentlichungstag und Bekanntmachung des Hinweises auf die Patenterteilung: **05.03.2008**
(21) Anmeldenummer: 04803851.7
(22) Anmeldetag: 14.12.2004
(51) Int. Cl.: C07D 487/04, A01N 43/90

(54) **6-(2-FLUOR-4-ALKOXYPHENYL)-TRIAZOLOPYRIMIDINE, VERFAHREN ZU IHRER HERSTELLUNG, IHRE VERWENDUNG ZUR BEKÄMPFUNG VON SCHADPILEN SOWIE SIE ENTHALTENDE MITTEL**
6-(2-FLUORO-4-ALKOXYPHENYL)-TRIAZOLOPYRIMIDINES, METHODS FOR THEIR PRODUCTION, THEIR USE AGAINST FUNGI AND AGENTS CONTAINING THEM
6-(2-FLUORO-4-ALCOXYPHENYL)-TRIAZOLOPYRIMIDINES, LEURS PROCEDES DE FABRICATION, LEUR UTILISATION POUR LUTTER CONTRE LES CHAMPIGNION NUISIBLES ET AGENTS LES CONTENANTS

(30) Priorität: 17.12.2003 DE 10359435
(43) Veröffentlichungstag der Anmeldung: 06.09.2006
(73) Patentinhaber: BASF SE, 67056 Ludwigshafen (DE)
(72) Erfinder: TORMO I BLASCO, Jordi, 69514 Laudenbach (DE); BLETTNER, Carsten, 68165 Mannheim (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); WAGNER, Oliver, 67433 Neustadt (DE); SCHERER, Maria, 76829 Godramstein (DE); STRATHMANN, Siegfried, 67117 Limburgerhof (DE); SCHÖFL, Ulrich, 68782 Brühl (DE); STIERL, Reinhard, 67251 Freinsheim (DE)
(74) Vertreter: Isenbruck, Günter
(86) Internationale Anmeldenummer: PCT/EP2004/014228
(87) Internationale Veröffentlichungsnummer: WO 2005/058905

(56) Entgegenhaltungen:
- WO-A-02/38565
- WO-A-99/48893
- WO-A-02/083677
- WO-A-03/008417
- US-A- 5 981 534
- US-A- 5 994 360
- US-B1- 6 204 269

## Beschreibung

Die vorliegende Erfindung betrifft 6-(2-Halogen-4-alkoxyphenyl)-triazolopyrimidine der Formel I 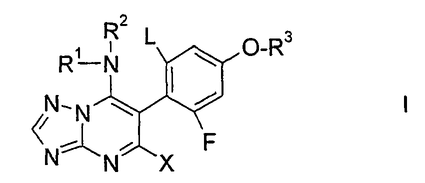 in der die Substituenten folgende Bedeutung haben:
- R¹: C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf-oder sechsgliedriger gesättigter, partiell ungesättigter oder aromatischer Heterocyclus, enthaltend ein bis vier Heteroatome aus der Gruppe O, N oder S,
- R²: Wasserstoff oder eine der bei R¹ genannten Gruppen,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
- R³: C₁-C₈-Alkyl, C₁-C₈-Halogenalkyl, C₃-C₈-Alkenyl, C₃-C₈-Halogenalkenyl, C₃-C₈-Alkinyl, C₃-C₈-Halogenalkinyl, Phenyl, Phenyl-C₁-C₄-alkyl, Mono- oder Di-(C₁-C₄-Alkoxy)-C₁-C₄-alkyl;
R¹, R² und/oder R³ können eine bis vier gleiche oder verschiedene Gruppen R^{a} tragen:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₈-Alkenyl, C₂-C₈-Halogenalkenyl, C₂-C₆-Alkenyloxy, C₂-C₈-Alkinyl, C₂-C₈-Halogenalkinyl, C₃-C₆-Alkinyloxy, Oxy-C₁-C₃-alkylenoxy, C₃-C₈-Cycloalkenyl, Phenyl, Naphthyl, fünf- oder sechsgliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein können;
- L: Wasserstoff, Fluor oder Chlor;
- X: Cyano, C₁-C₄-Alkyl oder C₁-C₄-Alkoxy

Außerdem betrifft die Erfindung ein Verfahren zur Herstellung dieser Verbindungen, sie enthaltende Mittel sowie ihre Verwendung zur Bekämpfung von pflanzenpathogenen Schadpilzen.

5-Halogen-6-(2-Halogen-4-alkoxyphenyl)-triazolopyrimidine sind aus WO 99/48893 allgemein bekannt. 5-Cyano- und 5-Alkoxy-triazolopyrimidine sind in WO 02/083677 offenbart. Triazolopyrimidine mit optisch aktiven Aminosubstituenten in 7-Position werden in WO 02/38565 allgemein vorgeschlagen.

Die in den vorgenannten Schriften beschriebenen Verbindungen sind zur Bekämpfung von Schadpilzen geeignet.

Ihre Wirkung ist jedoch nicht immer in jeder Hinsicht völlig zufriedenstellend. Davon ausgehend liegt der vorliegenden Erfindung die Aufgabe zugrunde, Verbindungen mit verbesserter Wirkung und/oder verbreitertem Wirkungsspektrum bereitzustellen.

Demgemäss wurden die eingangs definierten Verbindungen gefunden. Desweiteren wurde ein Verfahren zu ihrer Herstellung, sie enthaltende Mittel sowie Verfahren zur Bekämpfung von Schadpilzen unter Verwendung der Verbindungen I gefunden.

Die erfindungsgemäßen Verbindungen unterscheiden sich von den in der vorgenannten Schrift beschriebenen durch die Substitution in der 5-Position des Triazolopyrimidin-Gerüstes.

Die Verbindungen der Formel I weisen eine gegenüber den bekannten Verbindungen erhöhte Wirksamkeit, bzw. ein verbreitertes Wirkungsspektrum gegen Schadpilze auf.

Die erfindungsgemäßen Verbindungen können auf verschiedenen Wegen erhalten werden. Vorteilhaft werden sie ausgehend von den aus WO 99/48893 bekannten 5-Halogen-6-(2-Halogen-4-alkoxyphenyl)-triazolopyrimidinen der Formel II durch Umsetzung mit Verbindungen M-X (Formel III) erhalten. Verbindungen III stellen je nach der Bedeutung der einzuführenden Gruppe X ein anorganisches Cyanid oder ein Alkoxylat dar. Die Umsetzung erfolgt vorteilhaft in Anwesenheit eines inerten Lösungsmittels. Das Kation M in Formel III hat geringe Bedeutung; aus praktischen Gründen sind üblicherweise Ammonium-, Tetraalkylammonium- oder Alkali- oder Erdalkalimetallsalze bevorzugt. 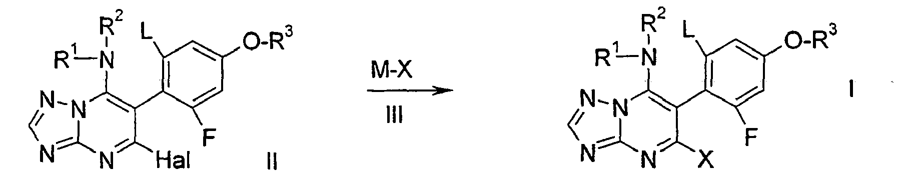 Üblicherweise liegt die Reaktionstemperatur bei 0 bis 120°C, bevorzugt bei 10 bis 40°C [vgl. J. Heterocycl. Chem., Bd.12, S. 861-863 (1975)].

Sofern R² Wasserstoff bedeutet, wird vorteilhaft vor Umsetzung mit III eine abspaltbare Schutzgruppe eingeführt [vgl. Greene, Protective Groups in Organic Chemistry, J. Wiley & Sons, (1981)].

Geeignete Lösungsmittel umfassen Ether, wie Dioxan, Diethylether und, bevorzugt Tetrahydrofuran, Alkohole, wie Methanol oder Ethanol, halogenierte Kohlenwasserstoffe wie Dichlormethan und aromatische Kohlenwasserstoffe, wie Toluol oder Acetonitril.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Verbindungen der Formel 1, in denen X für C₁-C₄-Alkyl steht, können vorteilhaft durch folgenden Syntheseweg erhalten werden:

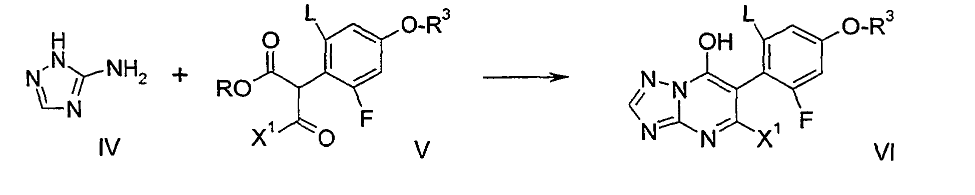

Ausgehend von 2-Aminotriazol IV und Ketoestern V werden die 5-Alkyl-7-hydroxy-6-phenyltriazolopyrimidine VI erhalten. In Formeln V und VI steht X¹ für C₁-C₄-Alkyl. Durch Verwendung der leicht zugänglichen 2-Phenylacetessigestern (V mit X¹=CH₃) werden die 5-Methyl-7-hydroxy-6-phenyltriazolopyrimidine erhalten, die einen bevorzugten Gegenstand der Erfindung darstellen [vgl. Chem. Pharm. Bull., 9, 801, (1961)]. 2-Aminotriazol IV ist kommerziell erhältlich. Die Herstellung der Ausgangsverbindungen Verfolgt vorteilhaft unter den aus EP-A 10 02 788 beschrieben Bedingungen.

Die so erhaltenen 5-Alkyl-7-hydroxy-6-phenyltriazolopyrimidine werden mit Halogenierungs-mitteln [HAL] unter den aus WO-A 94/20501 bekannten Bedingungen in die Halogenpyrimidine der Formel VII überführt, in der Hal ein Halogenatom, bevorzugt ein Brom oder ein Chloratom, insbesondere ein Chloratom bedeutet. Als Halogenierungsmittel [HAL] wird vorteilhaft ein Chlorierungs- oder Bromierungsmittel wie Phosphoroxybromid, Phosphoroxychlorid, Thionylchlorid, Thionylbromid oder Sulfurylchlorid eingesetzt.

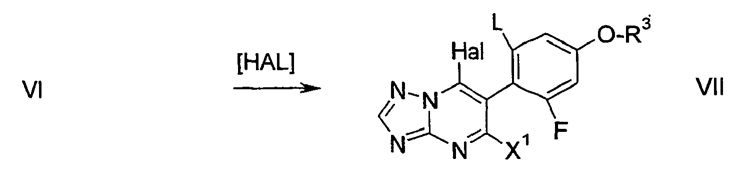

Diese Umsetzung wird üblicherweise bei 0°C bis 150°C, bevorzugt bei 80°C bis 125°C, durchgeführt [vgl. EP-A 770 615].

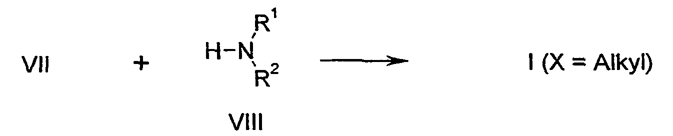

Die Umsetzung von VII mit Aminen VIII, wobei R¹ und R² wie in Formel I definiert sind, wird vorteilhaft bei 0°C bis 70°C, bevorzugt 10°C bis 35°C durchgeführt, vorzugsweise in Anwesenheit eines inerten Lösungsmittels, wie Ether, z. B. Dioxan, Diethylether oder insbesondere Tetrahydrofuran, halogenierte Kohlenwasserstoffe, wie Dichlormethan und aromatische Kohlenwasserstoffe, wie beispielsweise Toluol [vgl. WO-A 98/46608].

Die Verwendung einer Base, wie tertiäre Amine, beispielsweise Triethylamin oder anorganische Amine, wie Kaliumcarbonat ist bevorzugt; auch überschüssiges Amin der Formel VIII kann als Base dienen.

Verbindungen der Formel I, in der X C₁-C₄-Alkyl bedeutet, können alternativ auch aus Verbindungen I, in der X Halogen, insbesondere Chlor, bedeutet und Malonaten der Formel IX hergestellt werden. In Formel IX bedeuten X" Wasserstoff oder C₁-C₃-Alkyl und R C₁-C₄-Alkyl. Sie werden zu Verbindungen der Formel X umgesetzt und zu Verbindungen I decarboxyliert [vgl. US 5 994 360].

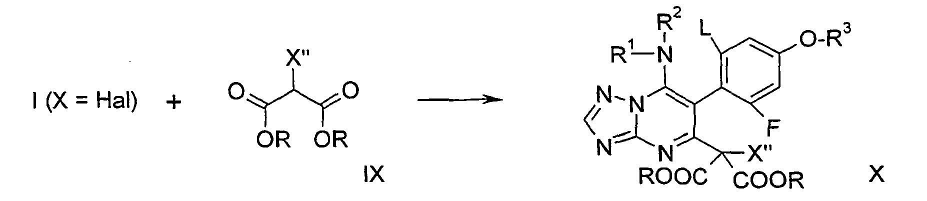

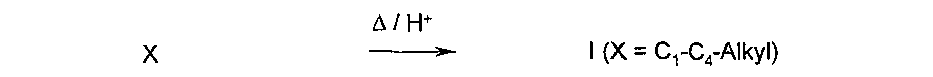

Die Malonate IX sind in der Literatur bekannt [J. Am. Chem. Soc., Bd. 64, 2714 (1942); J. Org. Chem., Bd. 39, 2172 (1974); Helv. Chim. Acta, Bd. 61,1565 (1978)] oder können gemäß der zitierten Literatur hergestellt werden.

Die anschließende Verseifung des Esters X erfolgt unter allgemein üblichen Bedingungen, in Abhängigkeit der verschiedenen Strukturelemente kann die alkalische oder die saure Verseifung der Verbindungen X vorteilhaft sein. Unter den Bedingungen der Esterverseifung kann die Decarboxylierung zu 1 bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I, in denen X für C₁-C₄-Alkyl steht, können auch durch Kupplung von 5-Halogentriazolopyrimidinen der Formel I, in der X Halogen bedeutet, mit metallorganischen Reagenzien der Formel XI erhalten werden. In einer Ausführungsform dieses Verfahrens erfolgt die Umsetzung unter Übergangsmetallkatalyse, wie Ni-oder Pd-Katalyse.

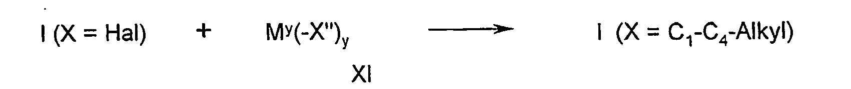

In Formel XI steht M für ein Metallion der Wertigkeit Y, wie beispielsweise B, Zn oder Sn und X" für C₁-C₃-Alkyl. Diese Reaktion kann beispielsweise analog folgender Methoden durchgeführt werden: J. Chem. Soc. Perkin Trans. 1, 1 187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure-oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Halogenalkyl: geradkettige oder verzweigte Alkylgruppen mit 1 bis 2, 4, 6 oder 8 Kohlenstoffatomen (wie vorstehend genannt), wobei in diesen Gruppen teilweise oder vollständig die Wasserstoffatome durch Halogenatome wie vorstehend genannt ersetzt sein können: insbesondere C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
Alkenyl: ungesättigte, geradkettige oder verzweigte Kohlenwasserstoffreste mit 2 bis 4, 6, 8 oder 10 Kohlenstoffatomen und einer oder zwei Doppelbindungen in beliebiger Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Halogenalkenyl: ungesättigte, geradkettige oder verzweigte Kohlenwasserstoffreste mit 2 bis 8 Kohlenstoffatomen und einer oder zwei Doppelbindungen in beliebiger Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
Alkinyl: geradkettige oder verzweigte Kohlenwasserstoffgruppen mit 2 bis 4, 6 oder 8 Kohlenstoffatomen und einer oder zwei Dreifachbindungen in beliebiger Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1, 2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: mono- oder bicyclische, gesättigte Kohlenwasserstoffgruppen mit 3 bis 6 oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
fünf- oder sechsgliedriger gesättigter, partiell ungesättigter oder aromatischer Heterocyclus, enthaltend ein bis vier Heteroatome aus der Gruppe O, N oder S:
   - 5- oder 6-gliedriges Heterocyclyl, enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;
Alkylen: gesättigte, geradkettige oder verzweigte Kohlenwasserstoffreste mit 1 bis 4 oder 6 Kohlenstoffatomen, welche über eine Doppelbindung an das Gerüst gebunden sind, z. B. =CH₂, =CH-CH₃, =CH-CH₂-CH₃;
Oxyalkylenoxy: divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

In dem Umfang der vorliegenden Erfindung sind die (R)- und (S)-Isomere und die Razemate von Verbindungen der Formel I eingeschlossen, die chirale Zentren aufweisen.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen I werden bevorzugt, in denen R¹ für C₁-C₄-Alkyl, C₂-C₆-Alkenyl oder C₁-C₈-Halogenalkyl steht.

Verbindungen 1 sind bevorzugt, in denen R¹ für eine Gruppe A steht: 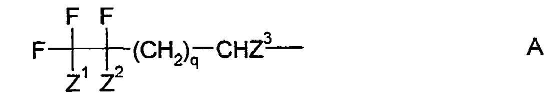 worin
- Z¹: Wasserstoff, Fluor oder C₁-C₆-Fluoroalkyl,
- Z²: Wasserstoff oder Fluor, oder Z¹ und Z² bilden gemeinsam eine Doppelbindung;
- q: 0 oder 1 ist; und
- Z³: Wasserstoff oder Methyl bedeuten.

Außerdem werden Verbindungen I bevorzugt, in denen R¹ für C₃-C₆-Cycloalkyl steht, welches durch C₁-C₄-Alkyl substituiert sein kann.

Insbesondere werden Verbindungen I bevorzugt, in denen R² Wasserstoff bedeutet.

Gleichermaßen bevorzugt sind Verbindungen 1, in denen R² für Methyl oder Ethyl steht.

Sofern R¹ und/oder R² Halogenalkyl oder Halogenalkenylgruppen mit Chiralitätszentrum beinhalten, sind für diese Gruppen die (S)- Isomere bevorzugt. Im Fall halogenfreier Alkyl oder Alkenylgruppen mit Chiralitätszentrum in R¹ oder R² sind die (R)-konfigurierten Isomere bevorzugt.

Eine bevorzugte Ausführungsform der Erfindung betrifft Verbindungen der Formel I.1: 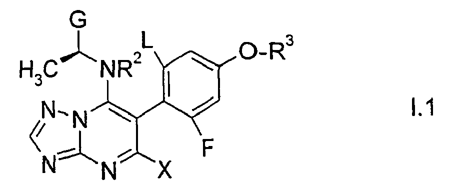 in der
- G: C₂-C₆-Alkyl, insbesondere Ethyl, n- und i-Propyl, n-, sek-, tert- Butyl, und C₁-C₄-Alkoxymethyl, insbesondere Ethoxymethyl, oder C₃-C₆-Cycloalkyl, insbesondere Cyclopentyl oder Cyclohexyl;
- R²: Wasserstoff oder Methyl;
- X, L und R³: wie eingangs definiert sind, wobei X insbesondere Cyano, Methoxy oder Ethoxy bedeuten.

Eine weitere bevorzugte Ausführungsform der Erfindung betrifft Verbindungen der Formel I.2. 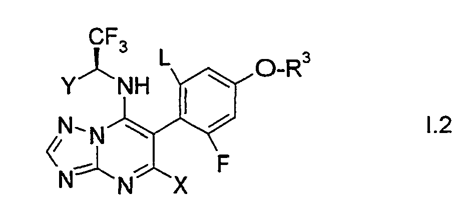 in der Y für Wasserstoff oder C₁-C₄-Alkyl, insbesondere für Methyl und Ethyl, und X, L und R³ wie eingangs definiert sind, wobei X insbesondere für Cyano, Methoxy oder Ethoxy steht.

Eine weitere bevorzugte Ausführungsform der Erfindung betrifft Verbindungen, in denen R¹ und R² zusammen mit dem Stickstoffatom, an das sie gebunden sind, ein fünf-oder sechsgliedriges Heterocyclyl oder Heteroaryl bilden, welches über N gebunden ist und ein weiteres Heteroatom aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, C₁-C₆-Alkylen und Oxy-C₁-C₃-alkylenoxy tragen kann. Diese Verbindungen entsprechen insbesondere Formel 1.3, 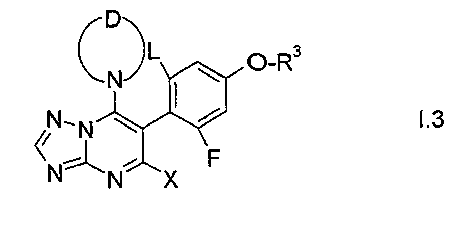 in der
- D: zusammen mit dem Stickstoffatom ein fünf- oder sechsgliedriges Heterocyclyl oder Heteroaryl bildet, welches über N gebunden ist und ein weiteres Heteroatom aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
- X, L und R³: wie eingangs definiert sind, wobei X insbesondere Cyano, Methoxy oder Ethoxy bedeuten.

Besonders bevorzugt sind Verbindungen der Formel 1.3, in der L Wasserstoff und R³ Methyl bedeuten.

Weiterhin werden Verbindungen I bevorzugt, in denen R¹ und R² zusammen mit dem Stickstoffatom, an das sie gebunden sind, einen Piperidinyl-, Morpholinyl- oder Thiomorpholinylring bilden, insbesondere einen Piperidinylring, der ggf. durch eine bis drei Gruppen Halogen, C₁-C₄-Alkyl oder C₁-C₄-Halogenalkyl substituiert ist. Besonders bevorzugt sind die Verbindungen, in denen R¹ und R² zusammen mit dem Stickstoffatom, an das sie gebunden sind, einen 4-Methylpiperidinring bilden.

Ein weiterer bevorzugter Gegenstand der Erfindung sind Verbindungen I, in denen R¹ und R² zusammen mit dem Stickstoffatom, an das sie gebunden sind, einen Pyrazolring bilden, der ggf. durch eine oder zwei Gruppen Halogen, C₁-C₄-Alkyl oder C₁-C₄-Halogenalkyl, insbesondere durch 3,5-Dimethyl oder 3,5-Di-(trifluormethyl) substituiert ist.

Ein besonders bevorzugter Erfindungsgegenstand sind Verbindungen der Formel I, in denen X Cyano, Methoxy oder Ethoxy, insbesondere Cyano oder Methoxy bedeutet.

In einer anderen bevorzugten Ausgestaltung der Verbindungen der Formel 1 bedeutet X C₁-C₄-Alkyl, insbesondere Methyl.

Daneben sind auch Verbindungen der Formel 1 besonders bevorzugt, in denen R¹CH(CH₃)-CH₂CH₃ , CH(CH₃)-CH(CH₃)₂ , CH(CH₃)-C(CH₃)₃, CH(CH₃)-CF₃ , CH₂C(CH₃)=CH₂ ,CH₂CH=CH₂, Cyclopentyl oder Cyclohexyl; R² Wasserstoff oder Methyl; oder R¹ und R² gemeinsam -(CH₂)₂CH(CH₃)(CH₂)₂-, -(CH₂)₂CH(CF₃)(CH₂)₂- oder -(CH₂)₂O(CH₂)₂- bedeuten, insbesondere solche, in denen X Cyano oder Methoxy bedeutet.

Ein weiterer bevorzugter Gegenstand sind Verbindungen der Formel I, in der R³ für Alkyl, insbesondere für Methyl steht.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

*Verbindungen* der Formel 1, in denen L Wasserstoff, R³ Methyl und X Cyano bedeutet und die Kombination von R¹ und R² für eine Verbindung jeweils einer Zeile der Tabelle A entspricht

### Tabelle 2

Verbindungen der Formel I, in denen L Fluor, R³ Methyl und X Cyano bedeutet und die Kombination von R¹ und R² für eine Verbindung jeweils einer Zeile der Tabelle A entspricht

### Tabelle 3

Verbindungen der Formel I, in denen L Chlor, R³ Methyl und X Cyano bedeutet und die Kombination von R¹ und R² für eine Verbindung jeweils einer Zeile der Tabelle A entspricht

### Tabelle 4

Verbindungen der Formel I, in denen L Wasserstoff, R³ Ethyl und X Cyano bedeutet und die Kombination von R¹ und R² für eine Verbindung jeweils einer Zeile der Tabelle A entspricht

### Tabelle 5

Verbindungen der Formel I, in denen L Fluor, R³ Ethyl und X Cyano bedeutet und die Kombination von R¹ und R² für eine Verbindung jeweils einer Zeile der Tabelle A entspricht

### Tabelle 6

Verbindungen der Formel I, in denen L Chlor, R³ Ethyl und X Cyano bedeutet und die Kombination von R¹ und R² für eine Verbindung jeweils einer Zeile der Tabelle A entspricht

### Tabelle 7

Verbindungen der Formel I, in denen L Wasserstoff, R³ n-Propyl und X Cyano bedeutet und die Kombination von R¹ und R² für eine Verbindung jeweils einer Zeile der Tabelle A entspricht

### Tabelle 8

Verbindungen der Formel 1, in denen L Fluor, R³ n-Propyl und X Cyano bedeutet und die Kombination von R¹ und R² für eine Verbindung jeweils einer Zeile der Tabelle A entspricht

### Tabelle 9

Verbindungen der Formel I, in denen L Chlor, R³ n-Propyl und X Cyano bedeutet und die Kombination von R¹ und R² für eine Verbindung jeweils einer Zeile der Tabelle A entspricht

### Tabelle 10

Verbindungen der Formel I, in denen L Wasserstoff, R³ iso-Propyl und X Cyano bedeutet und die Kombination von R¹ und R² für eine Verbindung jeweils einer Zeile der Tabelle A entspricht

### Tabelle 11

Verbindungen der Formel I, in denen L Fluor, R³ iso-Propyl und X Cyano bedeutet und die Kombination von R¹ und R² für eine Verbindung jeweils einer Zeile der Tabelle A entspricht

### Tabelle 12

Verbindungen der Formel I, in denen L Chlor, R³ iso-Propyl und X Cyano bedeutet und die Kombination von R¹ und R² für eine Verbindung jeweils einer Zeile der Tabelle A entspricht

### Tabelle 13

Verbindungen der Formel I, in denen L Wasserstoff, R³ 2-Fluorethyl und X Cyano bedeutet und die Kombination von R¹ und R² für eine Verbindung jeweils einer Zeile der Tabelle A entspricht

### Tabelle 14

Verbindungen der Formel I, in denen L Fluor, R³ 2-Fluorethyl und X Cyano bedeutet und die Kombination von R¹ und R² für eine Verbindung jeweils einer Zeile der Tabelle A entspricht

### Tabelle 15

Verbindungen der Formel I, in denen L Chlor, R³ 2-Fluorethyl und X Cyano bedeutet und die Kombination von R¹ und R² für eine Verbindung jeweils einer Zeile der Tabelle A entspricht

### Tabelle 16

Verbindungen der Formel I, in denen L Wasserstoff, R³ Allyl und X Cyano bedeutet und die Kombination von R¹ und R² für eine Verbindung jeweils einer Zeile der Tabelle A entspricht

### Tabelle 17

Verbindungen der Formel I, in denen L Fluor, R³ Allyl und X Cyano bedeutet und die Kombination von R¹ und R² für eine Verbindung jeweils einer Zeile der Tabelle A entspricht

### Tabelle 18

Verbindungen der Formel I, in denen L Chlor, R³ Allyl und X Cyano bedeutet und die Kombination von R¹ und R² für eine Verbindung jeweils einer Zeile der Tabelle A entspricht

### Tabelle 19

Verbindungen der Formel I, in denen L Wasserstoff, R³ 2-Methoxyethyl und X Cyano bedeutet und die Kombination von R¹ und R² für eine Verbindung jeweils einer Zeile der Tabelle A entspricht

### Tabelle 20

Verbindungen der Formel I, in denen L Fluor, R³ 2-Methoxyethyl und X Cyano bedeutet und die Kombination von R¹ und R² für eine Verbindung jeweils einer Zeile der Tabelle A entspricht

### Tabelle 21

Verbindungen der Formel I, in denen L Chlor, R³ 2-Methoxyethyl und X Cyano bedeutet und die Kombination von R¹ und R² für eine Verbindung jeweils einer Zeile der Tabelle A entspricht

### Tabelle 22

Verbindungen der Formel I, in denen L Wasserstoff, R³ Methyl und X Methoxy bedeutet und die Kombination von R¹ und R² für eine Verbindung jeweils einer Zeile der Tabelle A entspricht

### Tabelle 23

Verbindungen der Formel I, in denen L Fluor, R³ Methyl und X Methoxy bedeutet und die Kombination von R¹ und R² für eine Verbindung jeweils einer Zeile der Tabelle A entspricht

### Tabelle 24

Verbindungen der Formel I, in denen L Chlor, R³ Methyl und X Methoxy bedeutet und die Kombination von R¹ und R² für eine Verbindung jeweils einer Zeile der Tabelle A entspricht

### Tabelle 25

Verbindungen der Formel I, in denen L Wasserstoff, R³ Ethyl und X Methoxy bedeutet und die Kombination von R¹ und R² für eine Verbindung jeweils einer Zeile der Tabelle A entspricht

### Tabelle 26

Verbindungen der Formel 1, in denen L Fluor, R³ Ethyl und X Methoxy bedeutet und die Kombination von R¹ und R² für eine Verbindung jeweils einer Zeile der Tabelle A entspricht

### Tabelle 27

Verbindungen der Formel I, in denen L Chlor, R³ Ethyl und X Methoxy bedeutet und die Kombination von R¹ und R² für eine Verbindung jeweils einer Zeile der Tabelle A entspricht

### Tabelle 28

Verbindungen der Formel I, in denen L Wasserstoff, R³ n-Propyl und X Methoxy bedeutet und die Kombination von R¹ und R² für eine Verbindung jeweils einer Zeile der Tabelle A entspricht

### Tabelle 29

Verbindungen der Formel I, in denen L Fluor, R³ n-Propyl und X Methoxy bedeutet und die Kombination von R¹ und R² für eine Verbindung jeweils einer Zeile der Tabelle A entspricht

### Tabelle 30

Verbindungen der Formel I, in denen L Chlor, R³ n-Propyl und X Methoxy bedeutet und die Kombination von R¹ und R² für eine Verbindung jeweils einer Zeile der Tabelle A entspricht

### Tabelle 31

Verbindungen der Formel 1, in denen L Wasserstoff, R³ iso-Propyl und X Methoxy bedeutet und die Kombination von R¹ und R² für eine Verbindung jeweils einer Zeile der Tabelle A entspricht

### Tabelle 32

Verbindungen der Formel I, in denen L Fluor, R³ iso-Propyl und X Methoxy bedeutet und die Kombination von R¹ und R² für eine Verbindung jeweils einer Zeile der Tabelle A entspricht

### Tabelle 33

Verbindungen der Formel I, in denen L Chlor, R³ iso-Propyl und X Methoxy bedeutet und die Kombination von R¹ und R² für eine Verbindung jeweils einer Zeile der Tabelle A entspricht

### Tabelle 34

Verbindungen der Formel I, in denen L Wasserstoff, R³ 2-Fluorethyl und X Methoxy bedeutet und die Kombination von R¹ und R² für eine Verbindung jeweils einer Zeile der Tabelle A entspricht

### Tabelle 35

Verbindungen der Formel I, in denen L Fluor, R³ 2-Fluorethyl und X Methoxy bedeutet und die Kombination von R¹ und R² für eine Verbindung jeweils einer Zeile der Tabelle A entspricht

### Tabelle 36

Verbindungen der Formel 1, in denen L Chlor, R³ 2-Fluorethyl und X Methoxy bedeutet und die Kombination von R¹ und R² für eine Verbindung jeweils einer Zeile der Tabelle A entspricht

### Tabelle 37

Verbindungen der Formel I, in denen L Wasserstoff, R³ Allyl und X Methoxy bedeutet und die Kombination von R¹ und R² für eine Verbindung jeweils einer Zeile der Tabelle A entspricht

### Tabelle 38

Verbindungen der Formel I, in denen L Fluor, R³ Allyl und X Methoxy bedeutet und die Kombination von R¹ und R² für eine Verbindung jeweils einer Zeile der Tabelle A entspricht

### Tabelle 39

Verbindungen der Formel I, in denen L Chlor, R³ Allyl und X Methoxy bedeutet und die Kombination von R¹ und R² für eine Verbindung jeweils einer Zeile der Tabelle A entspricht

### Tabelle 40

Verbindungen der Formel I, in denen L Wasserstoff, R³ 2-Methoxyethyl und X Methoxy bedeutet und die Kombination von R¹ und R² für eine Verbindung jeweils einer Zeile der Tabelle A entspricht

### Tabelle 41

Verbindungen der Formel I, in denen L Fluor, R³ 2-Methoxyethyl und X Methoxy bedeutet und die Kombination von R¹ und R² für eine Verbindung jeweils einer Zeile der Tabelle A entspricht

### Tabelle 42

Verbindungen der Formel I, in denen L Chlor, R³ 2-Methoxyethyl und X Methoxy bedeutet und die Kombination von R¹ und R² für eine Verbindung jeweils einer Zeile der Tabelle A entspricht

### Tabelle 43

Verbindungen der Formel I, in denen L Wasserstoff, R³ Methyl und X Ethoxy bedeutet und die Kombination von R¹ und R² für eine Verbindung jeweils einer Zeile der Tabelle A entspricht

### Tabelle 44

Verbindungen der Formel I, in denen L Fluor, R³ Methyl und X Ethoxy bedeutet und die Kombination von R¹ und R² für eine Verbindung jeweils einer Zeile der Tabelle A entspricht

### Tabelle 45

Verbindungen der Formel I, in denen L Chlor, R³ Methyl und X Ethoxy bedeutet und die Kombination von R¹ und R² für eine Verbindung jeweils einer Zeile der Tabelle A entspricht

### Tabelle 46

Verbindungen der Formel 1, in denen L Wasserstoff, R³ Ethyl und X Ethoxy bedeutet und die Kombination von R¹ und R² für eine Verbindung jeweils einer Zeile der Tabelle A entspricht

### Tabelle 47

Verbindungen der Formel I, in denen L Fluor, R³ Ethyl und X Ethoxy bedeutet und die Kombination von R¹ und R² für eine Verbindung jeweils einer Zeile der Tabelle A entspricht

### Tabelle 48

Verbindungen der Formel I, in denen L Chlor, R³ Ethyl und X Ethoxy bedeutet und die Kombination von R¹ und R² für eine Verbindung jeweils einer Zeile der Tabelle A entspricht

### Tabelle 49

Verbindungen der Formel I, in denen L Wasserstoff, R³ n- Propyl- und X Ethoxy bedeutet und die Kombination von R¹ und R² für eine Verbindung jeweils einer Zeile der Tabelle A entspricht

### Tabelle 50

Verbindungen der Formel I, in denen L Fluor, R³ n-Propyl und X Ethoxy bedeutet und die Kombination von R¹ und R² für eine Verbindung jeweils einer Zeile der Tabelle A entspricht

### Tabelle 51

Verbindungen der Formel I, in denen L Chlor, R³ n-Propyl und X Ethoxy bedeutet und die Kombination von R¹ und R² für eine Verbindung jeweils einer Zeile der Tabelle A entspricht

### Tabelle 52

Verbindungen der Formel I, in denen L Wasserstoff, R³ iso-Propyl und X Ethoxy bedeutet und die Kombination von R¹ und R² für eine Verbindung jeweils einer Zeile der Tabelle A entspricht

### Tabelle 53

Verbindungen der Formel I, in denen L Fluor, R³ iso-Propyl und X Ethoxy bedeutet und die Kombination von R¹ und R² für eine Verbindung jeweils einer Zeile der Tabelle A entspricht

### Tabelle 54

Verbindungen der Formel I, in denen L Chlor, R³ iso-Propyl und X Ethoxy bedeutet und die Kombination von R¹ und R² für eine Verbindung jeweils einer Zeile der Tabelle A entspricht

### Tabelle 55

Verbindungen der Formel I, in denen L Wasserstoff, R³ 2-Fluorethyl und X Ethoxy bedeutet und die Kombination von R¹ und R² für eine Verbindung jeweils einer Zeile der Tabelle A entspricht

### Tabelle 56

Verbindungen der Formel I, in denen L Fluor, R³ 2-Fluorethyl und X Ethoxy bedeutet und die Kombination von R¹ und R² für eine Verbindung jeweils einer Zeile der Tabelle A entspricht

### Tabelle 57

Verbindungen der Formel I, in denen L Chlor, R³ 2-Fluorethyl und X Ethoxy bedeutet und die Kombination von R¹ und R² für eine Verbindung jeweils einer Zeile der Tabelle A entspricht

### Tabelle 58

Verbindungen der Formel I, in denen L Wasserstoff, R³ Allyl und X Ethoxy bedeutet und die Kombination von R¹ und R² für eine Verbindung jeweils einer Zeile der Tabelle A entspricht

### Tabelle 59

Verbindungen der Formel I, in denen L Fluor, R³ Allyl und X Ethoxy bedeutet und die Kombination von R¹ und R² für eine Verbindung jeweils einer Zeile der Tabelle A entspricht

### Tabelle 60

Verbindungen der Formel I, in denen L Chlor, R³ Allyl und X Ethoxy bedeutet und die Kombination von R¹ und R² für eine Verbindung jeweils einer Zeile der Tabelle A entspricht

### Tabelle 61

Verbindungen der Formel I, in denen L Wasserstoff, R³ 2-Methoxyethyl und X Ethoxy bedeutet und die Kombination von R¹ und R² für eine Verbindung jeweils einer Zeile der Tabelle A entspricht

### Tabelle 62

Verbindungen der Formel I, in denen L Fluor, R³ 2-Methoxyethyl und X Ethoxy bedeutet und die Kombination von R¹ und R² für eine Verbindung jeweils einer Zeile der Tabelle A entspricht

### Tabelle 63

Verbindungen der Formel 1, in denen L Chlor, R³ 2-Methoxyethyl und X Ethoxy bedeutet und die Kombination von R¹ und R² für eine Verbindung jeweils einer Zeile der Tabelle A entspricht

### Tabelle 64

Verbindungen der Formel I, in denen L Wasserstoff, R³ Methyl und X Methyl bedeutet und die Kombination von R¹ und R² für eine Verbindung jeweils einer Zeile der Tabelle A entspricht

### Tabelle 65

Verbindungen der Formel I, in denen L Fluor, R³ Methyl und X Methyl bedeutet und die Kombination von R¹ und R² für eine Verbindung jeweils einer Zeile der Tabelle A entspricht

### Tabelle 66

Verbindungen der Formel I, in denen L Chlor, R³ Methyl und X Methyl bedeutet und die Kombination von R¹ und R² für eine Verbindung jeweils einer Zeile der Tabelle A entspricht

### Tabelle 67

Verbindungen der Formel I, in denen L Wasserstoff, R³ Ethyl und X Methyl bedeutet und die Kombination von R¹ und R² für eine Verbindung jeweils einer Zeile der Tabelle A entspricht

### Tabelle 68

Verbindungen der Formel I, in denen L Fluor, R³ Ethyl und X Methyl bedeutet und die Kombination von R¹ und R² für eine Verbindung jeweils einer Zeile der Tabelle A entspricht

### Tabelle 69

Verbindungen der Formel I, in denen L Chlor, R³ Ethyl und X Methyl bedeutet und die Kombination von R¹ und R² für eine Verbindung jeweils einer Zeile der Tabelle A entspricht

### Tabelle 70

Verbindungen der Formel I, in denen L Wasserstoff, R³ n-Propyl und X Methyl bedeutet und die Kombination von R¹ und R² für eine Verbindung jeweils einer Zeile der Tabelle A entspricht

### Tabelle 71

Verbindungen der Formel I, in denen L Fluor, R³ n-Propyl und X Methyl bedeutet und die Kombination von R¹ und R² für eine Verbindung jeweils einer Zeile der Tabelle A entspricht

### Tabelle 72

Verbindungen der Formel I, in denen L Chlor, R³ n-Propyl und X Methyl bedeutet und die Kombination von R¹ und R² für eine Verbindung jeweils einer Zeile der Tabelle A entspricht

### Tabelle 73

Verbindungen der Formel I, in denen L Wasserstoff, R³ iso-Propyl und X Methyl bedeutet und die Kombination von R¹ und R² für eine Verbindung jeweils einer Zeile der Tabelle A entspricht

### Tabelle 74

Verbindungen der Formel I, in denen L Fluor, R³ iso-Propyl und X Methyl bedeutet und die Kombination von R¹ und R² für eine Verbindung jeweils einer Zeile der Tabelle A entspricht

### Tabelle 75

Verbindungen der Formel I, in denen L Chlor, R³ iso-Propyl und X Methyl bedeutet und die Kombination von R¹ und R² für eine Verbindung jeweils einer Zeile der Tabelle A entspricht

### Tabelle 76

Verbindungen der Formel I, in denen L Wasserstoff, R³ 2-Fluorethyl und X Methyl bedeutet und die Kombination von R¹ und R² für eine Verbindung jeweils einer Zeile der Tabelle A entspricht

### Tabelle 77

Verbindungen der Formel I, in denen L Fluor, R³ 2-Fluorethyl und X Methyl bedeutet und die Kombination von R¹ und R² für eine Verbindung jeweils einer Zeile der Tabelle A entspricht

### Tabelle 78

Verbindungen der Formel I, in denen L Chlor, R³ 2-Fluorethyl und X Methyl bedeutet und die Kombination von R¹ und R² für eine Verbindung jeweils einer Zeile der Tabelle A entspricht

### Tabelle 79

Verbindungen der Formel I, in denen L Wasserstoff, R³ Allyl und X Methyl bedeutet und die Kombination von R¹ und R² für eine Verbindung jeweils einer Zeile der Tabelle A entspricht

### Tabelle 80

Verbindungen der Formel I, in denen L Fluor, R³ Allyl und X Methyl bedeutet und die Kombination von R¹ und R² für eine Verbindung jeweils einer Zeile der Tabelle A entspricht

### Tabelle 81

Verbindungen der Formel 1, in denen L Chlor, R³ Allyl und X Methyl bedeutet und die Kombination von R¹ und R² für eine Verbindung jeweils einer Zeile der Tabelle A entspricht

### Tabelle 82

Verbindungen der Formel I, in denen L Wasserstoff, R³ 2-Methoxyethyl und X Methyl bedeutet und die Kombination von R¹ und R² für eine Verbindung jeweils einer Zeile der Tabelle A entspricht

### Tabelle 83

Verbindungen der Formel 1, in denen L Fluor, R³ 2-Methoxyethyl und X Methyl bedeutet und die Kombination von R¹ und R² für eine Verbindung jeweils einer Zeile der Tabelle A entspricht

### Tabelle 84

Verbindungen der Formel 1, in denen L Chlor, R³ 2-Methoxyethyl und X Methyl bedeutet und die Kombination von R¹ und R² für eine Verbindung jeweils einer Zeile der Tabelle A entspricht

**Tabelle A**

| **Nr.** | **R¹** | **R²** |
|---|---|---|
| A-1 | H | H |
| A-2 | CH₃ | H |
| A-3 | CH₃ | CH₃ |
| A-4 | CH₂CH₃ | H |
| A-5 | CH₂CH₃ | CH₃ |
| A-6 | CH₂CH₃ | CH₂CH₃ |
| A-7 | CH₂CF₃ | H |
| A-8 | CH₂CF₃ | CH₃ |
| A-9 | CH₂CF₃ | CH₂CH₃ |
| A-10 | CH₂CCl₃ | H |
| A-11 | CH₂CCl₃ | CH₃ |
| A-12 | CH₂CCl₃ | CH₂CH₃ |
| A-13 | CH₂CH₂CH₃ | H |
| A-14 | CH₂CH₂CH₃ | CH₃ |
| A-15 | CH₂CH₂CH₃ | CH₂CH₃ |
| A-16 | CH₂CH₂CH₃ | CH₂CH₂CH₃ |
| A-17 | CH(CH₃)₂ | H |
| A-18 | CH(CH₃)₂ | CH₃ |
| A-19 | CH(CH₃)₂ | CH₂CH₃ |
| A-20 | CH₂CH₂CH₂CH₃ | H |
| A-21 | CH₂CH₂CH₂CH₃ | CH₃ |
| A-22 | CH₂CH₂CH₂CH₃ | CH₂CH₃ |
| A-23 | CH₂CH₂CH₂CH₃ | CH₂CH₂CH₃ |
| A-24 | CH₂CH₂CH₂CH₃ | CH₂CH₂CH₂CH₃ |
| A-25 | (±) CH(CH₃)-CH₂CH₃ | H |
| A-26 | (±) CH(CH₃)-CH₂CH₃ | CH₃ |
| A-27 | (±) CH(CH₃)-CH₂CH₃ | CH₂CH₃ |
| A-28 | (S) CH(CH₃)-CH₂CH₃ | H |
| A-29 | (S) CH(CH₃)-CH₂CH₃ | CH₃ |
| A-30 | (S) CH(CH₃)-CH₂CH₃ | CH₂CH₃ |
| A-31 | (R) CH(CH₃)-CH₂CH₃ | H |
| A-32 | (R) CH(CH₃)-CH₂CH₃ | CH₃ |
| A-33 | (R) CH(CH₃)-CH₂CH₃ | CH₂CH₃ |
| A-34 | (±) CH(CH₃)-CH(CH₃)₂ | H |
| A-35 | (±) CH(CH₃)-CH(CH₃)₂ | CH₃ |
| A-36 | (±) CH(CH₃)-CH(CH₃)₂ | CH₂CH₃ |
| A-37 | (S) CH(CH₃)-CH(CH₃)₂ | H |
| A-38 | (S) CH(CH₃)-CH(CH₃)₂ | CH₃ |
| A-39 | (S) CH(CH₃)-CH(CH₃)₂ | CH₂CH₃ |
| A-40 | (R) CH(CH₃)-CH(CH₃)₂ | H |
| A-41 | (R) CH(CH₃)-CH(CH₃)₂ | CH₃ |
| A-42 | (R) CH(CH₃)-CH(CH₃)₂ | CH₂CH₃ |
| A-43 | (±) CH(CH₃)-C(CH₃)₃ | H |
| A-44 | (±) CH(CH₃)-C(CH₃)₃ | CH₃ |
| A-45 | (±) CH(CH₃)-C(CH₃)₃ | CH₂CH₃ |
| A-46 | (S) CH(CH₃)-C(CH₃)₃ | H |
| A-47 | (S) CH(CH₃)-C(CH₃)₃ | CH₃ |
| A-48 | (S) CH(CH₃)-C(CH₃)₃ | CH₂CH₃ |
| A-49 | (R) CH(CH₃)-C(CH₃)₃ | H |
| A-50 | (R) CH(CH₃)-C(CH₃)₃ | CH₃ |
| A-51 | (R) CH(CH₃)-C(CH₃)₃ | CH₂CH₃ |
| A-52 | (±) CH(CH₃)-CF₃ | H |
| A-53 | (±) CH(CH₃)-CF₃ | CH₃ |
| A-54 | (±) CH(CH₃)-CF₃ | CH₂CH₃ |
| A-55 | (S) CH(CH₃)-CF₃ | H |
| A-56 | (S) CH(CH₃)-CF₃ | CH₃ |
| A-57 | (S) CH(CH₃)-CF₃ | CH₂CH₃ |
| A-58 | (R) CH(CH₃)-CF₃ | H |
| A-59 | (R) CH(CH₃)-CF₃ | CH₃ |
| A-60 | (R) CH(CH₃)-CF₃ | CH₂CH₃ |
| A-61 | (±) CH(CH₃)-CCl₃ | H |
| A-62 | (±) CH(CH₃)-CCl₃ | CH₃ |
| A-63 | (±) CH(CH₃)-CCl₃ | CH₂CH₃ |
| A-64 | (S) CH(CH₃)-CCl₃ | H |
| A-65 | (S) CH(CH₃)-CCl₃ | CH₃ |
| A-66 | (S) CH(CH₃)-CCl₃ | CH₂CH₃ |
| A-67 | (R) CH(CH₃)-CCl₃ | H |
| A-68 | (R) CH(CH₃)-CCl₃ | CH₃ |
| A-69 | (R) CH(CH₃)-CCl₃ | CH₂CH₃ |
| A-70 | CH₂CF₂CF₃ | H |
| A-71 | CH₂CF₂CF₃ | CH₃ |
| A-72 | CH₂CF₂CF₃ | CH₂CH₃ |
| A-73 | CH₂(CF₂)₂CF₃ | H |
| A-74 | CH₂(CF₂)₂CF₃ | CH₃ |
| A-75 | CH₂(CF₂)₂CF₃ | CH₂CH₃ |
| A-76 | CH₂C(CH₃)=CH₂ | H |
| A-77 | CH₂C(CH₃)=CH₂ | CH₃ |
| A-78 | CH₂C(CH₃)₌CH₂ | CH₂CH₃ |
| A-79 | CH₂CH=CH₂ | H |
| A-80 | CH₂CH=CH₂ | CH₃ |
| A-81 | CH₂CH=CH₂ | CH₂CH₃ |
| A-82 | CH₂-C≡CH | H |
| A-83 | CH₂-C≡CH | CH₃ |
| A-84 | CH₂-C≡CH | CH₂CH₃ |
| A-85 | Cyclopentyl | H |
| A-86 | Cyclopentyl | CH₃ |
| A-87 | Cyclopentyl | CH₂CH₃ |
| A-88 | Cyclohexyl | H |
| A-89 | Cyclohexyl | CH₃ |
| A-90 | Cyclohexyl | CH₂CH₃ |
| A-91 | CH₂-C₆H₅ | H |
| A-92 | CH₂-C₆H₅ | CH₃ |
| A-93 | CH₂-C₆H₅ | CH₂CH₃ |
| A-94 | -(CH₂)₂CH=CHCH₂- | |
| A-95 | -(CH₂)₂C(CH₃)=CHCH₂- | |
| A-96 | -(CH₂)₂CH(CH₃)(CH₂)₂- | |
| A-97 | -(CH₂)₃CHFCH₂- | |
| A-98 | -(CH₂)₂CHF(CH₂)₂- | |
| A-99 | -CH₂CHF(CH₂)₃- | |
| A-100 | -(CH₂)₂CH(CF₃)(CH₂)₂- | |
| A-101 | -(CH₂)₂O(CH₂)₂- | |
| A-102 | -(CH₂)₂S(CH₂)₂- | |
| A-103 | -(CH₂)₅- | |
| A-104 | -(CH₂)₄- | |
| A-105 | -CH₂CH=CHCH₂- | |
| A-106 | -CH(CH₃)(CH₂)₃- | |
| A-107 | -CH₂CH(CH₃)(CH₂)₂- | |
| A-108 | -CH(CH₃)-(CH₂)₂-CH(CH₃)- | |
| A-109 | -CH(CH₃)-(CH₂)₄- | |
| A-110 | -CH₂-CH(CH₃)-(CH₂)₃- | |
| A-111 | -(CH₂)-CH(CH₃)-CH₂-CH(CH₃)-CH₂- | |
| A-112 | -CH(CH₂CH₃)-(CH₂)₄- | |
| A-113 | -(CH₂)₂-CHOH-(CH₂)₂- | |
| A-114 | -(CH₂)-CH=CH-(CH₂)₂- | |
| A-115 | -(CH₂)₆- | |
| A-116 | -CH(CH₃)-(CH₂)₅- | |
| A-117 | -(CH₂)₂-N(CH₃)-(CH₂)₂- | |
| A-118 | -N=CH-CH=CH- | |
| A-119 | -N=C(CH₃)-CH=C(CH₃)- | |
| A-120 | -N=C(CF₃)-CH=C(CF₃)- | |

Die Verbindungen I eignen sich als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.
Speziell eignen sie sich zur Bekämpfung folgender Pflanzenkrankheiten:
- *Alternaria-*Arten an Gemüse und Obst,
- *Bipolaris-* und *Drechslera-*Arten an Getreide, Reis und Rasen,
- *Blumeria graminis* (echter Mehltau) an Getreide,
- *Botrytis cinerea* (Grauschimmel) an Erdbeeren, Gemüse, Zierpflanzen und Reben,
- *Erysiphe cichoracearum* und *Sphaerotheca fuliginea* an Kürbisgewächsen,
- *Fusarium-* und *Verticillium-Arten* an verschiedenen Pflanzen,
- *Mycosphaerella-*Arten an Getreide, Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora-*Arten an Hopfen und Gurken,
- *Puccinia-*Arten an Getreide,
- *Pyricularia oryzae* an Reis,
- *Rhizoctonia-*Arten an Baumwolle, Reis und Rasen,
- *Septoria tritici* und *Stagonospora nodorum* an Weizen,
- *Uncinula necator* an Reben,
- *Ustilago-*Arten an Getreide und Zuckerrohr, sowie
- *Venturia-*Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen 1 werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 1 bis 1000 g/100 kg, vorzugsweise 5 bis 100 g je 100 Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
- Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie LigninSulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser

### A Wasserlösliche Konzentrate (SL)

10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.

### B Dispergierbare Konzentrate (DC)

20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.

### C Emulgierbare Konzentrate (EC)

15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.

### D Emulsionen (EW, EO)

40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.

### E Suspensionen (SC, OD)

20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.

### F Wasserdispergierbare und wasserlösliche Granulate (WG, SG)

50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.

### G Wasserdispergierbare und wasserlösliche Pulver (WP, SP)

75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

### H Stäube (DP)

5 Gew.Teile einer erfindungsgemäßen Verbindung werden fein gemahlen und mit 95 % feinteiligem Kaolin innig vermischt. Man erhält dadurch ein Stäubemittel.

### 1 Granulate (GR, FG, GG, MG)

0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.

### J ULV- Lösungen (UL)

10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Enilconazol, Epoxiconazol, Fenbuconazol, Fluquiconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
- Dicarboximide wie lprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthal-isopropyl
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid
- Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 5-Cyano-6-(2,6-difluor,4-methoxy-phenyl)-7-(4-methylpiperidinyl)-1,2,4-triazolo[1,5a]pyrimidin

Eine Lösung von 0,2 g (0,51 mmol) 5-Chlor-6-(2,6-difluor,4-methoxy-phenyl)-7-(4-methylpiperidinyl)-1,2,4-triazolo[1,5a]pyrimidin (vgl. EP-A 550 113) und 0,4 g (1,5 mmol) Tetrabutylammoniumcyanid in 10 ml Acetonitril wurde etwa 14 Std. bei 20-25°C und anschließend 5 Std. bei 45°C gerührt. Anschließend wurde die Reaktionsmischung über Kieselgel abfiltriert, das Filtrat wurde vom Lösungsmittel befreit. Der Rückstand wurde mittels präparativer MPLC über Kieselgel RP-18 (Eluent Acetonitril-Wasser) gereinigt. Man erhielt 0,06 g der Titelverbindung als farblosen Festkörper vom Fp 218°C.
¹H-NMR (CDCl₃, δ in ppm): 8,5 (s, 1H); 6,55 (d, 2H); 3,9 (s, 3H); 3,8 (d, 2H); 2,9 (t, 2H); 1,7 (d, 2H); 1,6 (m, 1 H); 1,4 (m, 2H); 0,95 (d, 3H)

### Beispiel 2: Herstellung von 5-Methyl-6-(2,6-difluor,4-methoxy-phenyl)-7-(4-methylpiperidinyl)-1,2,4-triazolo[1,5a]pyrimidin

Eine Lösung von 1 g (2,5 mmol) 5-Chlor-6-(2,6-difluor,4-methoxy-phenyl)-7-(4-methylpiperidinyl)-1,2,4-triazolo[1,5a]pyrimidin (vgl. EP-A 550 113) und 1 g (6,3 mmol) Natrium-dimethylmalonat in 10 ml Acetonitril wurde ca. 3 Std. bei 70-80°C gerührt. Anschließend gab man nochmals 1 g Natrium-dimethylmalonat hinzu und rührte weitere 3 Std. bei 70-80°C. Dabei fiel ein gelber Niederschlag aus. Anschließend filtrierte man die Reaktionsmischung über Kieselgur, nahm den gelben Niederschlag in Methylenchlorid und verdünnter Salzsäure auf und rührte ca. 15 min bei 20-25°C. Nach Phasentrennung wurde die organische Phase getrocknet und vom Lösungsmittel befreit. Der erhaltene Rückstand wurde in 30 ml konz. Salzsäure aufgenommen und die Reaktionsmischung wurde 5 Std. refluxiert. Anschließend gab man die Reaktionsmischung auf Eiswasser und extrahierte die wässrige Phase mit Methylenchlorid. Die vereinigten organischen Phasen wurden getrocknet und vom Lösungsmittel befreit. Man erhielt 0,45 g der Titelverbindung als hellen Festkörper vom Fp 83°C.
¹H-NMR (CDCl₃, δ in ppm): 8,4 (s, 1 H); 6,6 (d, 2H); 3,9 (s, 3H); 3,6 (d, 2H); 2,75 (t, 2H); 2,4 (s, 3H); 1,6 (d, 2H); 1,5 (m, 1H); 1,3 (m, 2H); 0,95 (d, 3H)

### Beispiel 3: Herstellung von 5-Methoxy-6-(2,6-difluor,4-methoxy-phenyl)-7-(4-methylpiperidinyl)-1,2,4-triazolo[1,5a]pyrimidin

Eine Lösung von 0,2 g (0,51 mmol) 5-Chlor-6-(2,6-difluor,4-methoxy-phenyl)-7-(4-methylpiperidinyl)-1,2,4-triazolo[1,5a]pyrimidin (vgl. EP-A 550 113) und 1,5 g 30 %ige Natriummethylat-Lsg in 5 ml Methanol wurde etwa 14 Std. bei 20-25°C gerührt. Die Reaktionsmischung wurde über Kieselgel abfiltriert und vom Lösungsmittel befreit. Der Rückstand wurde mittels präparativer MPLC über Kieselgel RP-18 (Eluent Acetonitril-Wasser) gereinigt. Man erhielt 0,12 g der Titelverbindung als gelbes Harz.
¹H-NMR (CDCl₃, δ in ppm): 8,25 (s, 1H); 6,6 (d, 2H); 4,0 (s, 3H); 3,85 (s, 3H); 3,65 (d, 2H); 2,75 (t, 2H); 1,6 (d, 2H); 1,5 (m, 1 H); 1,35 (m, 2H); 0,95 (d, 3H)

**Tabelle 1 - Verbindungen der Formel 1:**

| Nr. | R¹ | R² | R³ | L | X | Phys. Daten Fp [°C] |
|---|---|---|---|---|---|---|
| I-1 | -(CH₂)₂CH(CH₃)(CH₂)₂- | | CH₃ | F | -CN | 218 |
| I-2 | -(CH₂)₂CH(CH₃)(CH₂)₂- | | CH₃ | F | -CH₃ | 83 |
| I-3 | -(CH₂)₂CH(CH₃)(CH₂)₂- | | CH₃ | F | -OCH₃ | (s. Bsp. 3) |
| I-4 | -CH(CH₃)₂ | H | CH₃ | Cl | -OCH₃ | 169 |
| I-5 | -(CH₂)₂CH(CH₃)(CH₂)₂- | | n-Butyl | F | -O-n-Butyl | Harz |

### Beispiele für die Wirkung gegen Schadpilze

Die fungizide Wirkung der Verbindungen der Formel 1 ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Wettol EM 31 (Netzmittel mit Emulgier- und Dispergierwirkung auf der Basis ethoxylierter Alkylphenole) zugesetzt. Die Stammlösungen der Wirkstoffe wurden entsprechend der angegebenen Konzentration mit Wasser verdünnt.

Die Wirkstoffe wurden für die Beispiele 1 und 2 als eine Stammlösung aufbereitet mit 25 mg Wirkstoff, welcher mit einem Gemisch aus Aceton und/oder DMSO und dem Emulgator Uniperol^{®} EL (Netzmittel mit Emulgier- und Dispergierwirkung auf der Basis ethoxylierter Alkylphenole) im Volumen-Verhältnis Lösungsmittel-Emulgator von 99 zu 1 ad 10 ml aufgefüllt wurde. Anschließend wurde ad 100 ml mit Wasser aufgefüllt. Diese Stammlösung wurde mit dem beschriebenen Lösungsmittel-Emulgator-Wasser Gemisch zu der unten angegeben Wirkstoffkonzentration verdünnt.

### Anwendungsbeispiel 1 - Wirksamkeit gegen die Netzfleckenkrankheit der Gerste verursacht durch Pyrenophora teres bei 1 Tag protektiver Anwendung

Blätter von in Töpfen gewachsenen Gerstenkeimlingen der Sorte "Hanna" wurden mit wässriger Suspension in der unten angegebenen Wirkstoffkonzentration bis zur Tropfnässe besprüht. 24 Stunden nach dem Antrocknen des Spritzbelages wurden die Versuchspflanzen mit einer wässrigen Sporensuspension von *Pyrenophora [syn. Drechslera] teres,* dem Erreger der Netzfleckenkrankheit inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

In diesem Test zeigten die mit 250 ppm der Verbindungen I-1, I-2, bzw. I-3 behandelten Pflanzen nicht über 1 % Befall, während die unbehandelten Pflanzen zu 80 % befallen waren.

### Anwendungsbeispiel 2 - Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 2 bis 3 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1,7 × 10⁶ Sporen/ml in einer 2 %igen wässrigen Biomalzlösung enthielt, inokuliert. Anschließend wurden die Versuchspflanzen in eine Klimakammer mit 22 bis 24°C, Dunkelheit und hoher Luftfeuchtigkeit gestellt. Nach 5 Tagen konnte das Ausmaß des Pilzbefalls auf den Blättern visuell in % ermittelt werden.

In diesem Test zeigten die mit 250 ppm der Verbindungen I-1, I-2, bzw. I-3 behandelten Pflanzen keinen Befall, während die unbehandelten Pflanzen zu 80 % befallen waren.

### Anwendungsbeispiel 3 - Wirksamkeit gegen Altemaria solani an Tomaten (protektiv)

Blätter von Tomatenpflanzen der Sorte "Pixie II", die in Töpfen bis zum 4-Blattstadium kultiviert worden waren, wurden mit wässriger Wirkstoffaufbereitung, die mit einer Stammlösung aus 5 % Wirkstoff, 94 % Aceton und 1 % Emulgiermittel (Tween 20) angesetzt wurde, bis zur Tropfnässe besprüht. Nach dem Antrocknen des Spritzbelages (3-5 Std.) wurden die Blätter mit einer wässrigen Sporensuspension von *Alternaria solani* inokuliert (Dichte 15 × 10³ Sporen per ml). Anschließend wurden die Versuchspflanzen in Klimakammern bei 22 bis 24°C und 96 bis 99 % relativer Luftfeuchtigkeit für 36 Stunden aufgestellt und anschließend im Gewächshaus bei 21 bis 23°C und ungefähr 95 % relativer Luftfeuchtigkeit für weitere 2 bis 3 Tage kultiviert. Dann wurde das Ausmaß der Befallsentwicklung auf den Blättern visuell ermittelt.

In diesem Test zeigten die mit 250 ppm der Verbindung I-4 behandelten Pflanzen keinen Befall, während die unbehandelten Pflanzen zu 100 % befallen waren.

## Patentansprüche

1. 6-(2-Fluor-4-alkoxyphenyl)-triazolopyrimidine der Formel I 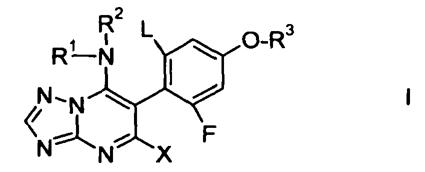 in der die Substituenten folgende Bedeutung haben:
R¹ C₁-C₈-Alkyl, C₁-C₈-Halogenalkyl, C₃-C₈-Cycloalkyl, C₃-C₈-Halogencycloalkyl, C₂-C₈-Alkenyl, C₂-C₈-Halogenalkenyl, C₃-C₆-Cycloalkenyl, C₃-C₆-Halogencycloalkenyl, C₂-C₈-Alkinyl, C₂-C₈-Halogenalkinyl oder Phenyl, Naphthyl, oder ein fünf- oder sechsgliedriger gesättigter, partiell ungesättigter oder aromatischer Heterocyclus, enthaltend ein bis vier Heteroatome aus der Gruppe O, N oder S,
R² Wasserstoff oder eine der bei R¹ genannten Gruppen,
R¹ und R² können auch zusammen mit dem Stickstoffatom, an das sie gebunden sind, ein fünf- oder sechsgliedriges Heterocyclyl oder Heteroaryl bilden, welches über N gebunden ist und ein bis drei weitere Heteroatome aus der Gruppe O, N und S als Ringglied enthalten und/oder einen oder mehrere Substituenten aus der Gruppe Halogen, C₁-C₆-Alkyl, C₁-C₆-Halogenalkyl, C₂-C₆-Alkenyl, C₂-C₆-Halogenalkenyl, C₁-C₆-Alkoxy, C₁-C₆-Halogenalkoxy, C₃-C₆-Alkenyloxy, C₃-C₆-Halogenalkenyloxy, (exo)-C₁-C₆-Alkylen und Oxy-C₁-C₃-alkylenoxy tragen kann;
R³ C₁-C₈-Alkyl, C₁-C₈-Halogenalkyl, C₃-C₈-Alkenyl, C₃-C₈-Halogenalkenyl, C₃-C₈-Alkinyl, C₃-C₈-Halogenalkinyl, Phenyl, Phenyl-C₁-C₄-alkyl, Monooder Di-(C₁-C₄-Alkoxy)-C₁-C₄-alkyl;
R¹, R² und/oder R³ können eine bis vier gleiche oder verschiedene Gruppen R^{a} tragen:
R^{a} Halogen, Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, DiC₁-C₆-alkylamino, C₂-C₈-Alkenyl, C₂-C₈-Halogenalkenyl, C₂-C₆-Alkenyloxy, C₂-C₈-Alkinyl, C₂-C₈-Halogenalkinyl, C₃-C₆-Alkinyloxy, Oxy-C₁-C₃-alkylenoxy, C₃-C₈-Cycloalkenyl, Phenyl, Naphthyl, fünf-oder sechsgliedriger gesättigter, partiell ungesättigter oder aromatischer Heterocyclus, enthaltend ein bis vier Heteroatome aus der Gruppe O, N oder S, wobei diese aliphatischen, alicyclischen oder aromatischen Gruppen ihrerseits partiell oder vollständig halogeniert sein können;
L Wasserstoff, Fluor oder Chlor; und
X Cyano, C₁-C₄-Alkyl, oder C₁-C₄-Alkoxy,

2. Verbindungen der Formel I gemäß Anspruch 1, in der X cyano oder C₁-C₄-Alkoxy, bedeutet.

3. Verbindungen der Formel I gemäß Anspruch 1 oder 2, in der X Cyano bedeutet.

4. Verbindungen der Formel I gemäß Anspruch 1, in der X Methyl bedeutet.

5. Verbindungen der Formel I gemäß Anspruch 1 oder 2, in der X Methoxy bedeutet.

6. Verbindungen der Formel I gemäß einem der Ansprüche 1 bis 5, in der R¹ und R² folgende Bedeutung haben:
R¹ CH(CH₃)-CH₂CH₃, CH(CH₃)-CH(CH₃)₂, CH(CH₃)-C(CH₃)₃ , CH(CH₃)-CF₃, CH₂C(CH₃)=CH₂ ,CH₂CH=CH₂, Cyclopentyl , Cyclohexyl;
R² Wasserstoff oder Methyl; oder
R¹ und R² bilden gemeinsam -(CH₂)₂CH(CH₃)(CH₂)₂-, -(CH₂)₂CH(CF₃)(CH₂)₂-oder -(CH₂)₂O(CH₂)₂-.

7. Verbindungen der Formel I. gemäß Anspruch 1 oder 2
in der
R¹ eine Gruppe 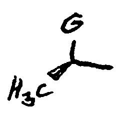 bedeutet,
G C₂-C₆-Alkyl, C₁-C₄-Alkoxymethyl oder C₃-C₆-Cycloalkyl;
R² Wasserstoff oder Methyl; und
X Cyano, Methyl, Methoxy oder Ethoxy bedeuten und
L und R³ gemäß Anspruch 1 definiert sind.

8. Verbindungen der Formel I gemäß Anspruch 1 oder 2,
R¹ eine Gruppe 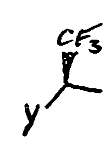 ist,
in der Y für Wasserstoff oder C₁-C₄-Alkyl und X für Cyano, Methyl, Methoxy oder Ethoxy steht und L und R³ gemäß Anspruch 1 definiert sind.

9. Verbindungen der Formel I gemäß Anspruch 1 oder 2,
in der R¹ und R² zusammen mit dem Stickstoff und D einen heterocyclischen Ring bilden, wobei
D zusammen mit dem Stickstoffatom ein fünf- oder sechsgliedriges Heterocyclyl oder Heteroaryl bildet, welches über N gebunden ist und ein weiteres Heteroatom aus der Gruppe O, N und S als Ringglied enthalten und/oder einen oder mehrere Substituenten aus der Gruppe Halogen, C₁-C₄-Alkyl, C₁-C₄-Alkoxy und C₁-C₂-Halogenalkyl tragen kann;
X Cyano, Methyl, Methoxy oder Ethoxy bedeuten und
L und R³ gemäß Anspruch 1 definiert sind.

10. Verbindungen gemäß Anspruch 9, in denen L Wasserstoff und R³ Methyl bedeuten.

11. Verbindungen der Formel I, gemäß einem der Ansprüche 1 bis 9, in denen L Fluor und R³ Methyl bedeuten.

12. Verfahren zur Herstellung der Verbindungen der Formel I gemäß Anspruch 2, durch Umsetzung von 5-Halogen-6-(2-Halogen-4-alkoxyphenyl)-triazolopyrimidinen der Formel II 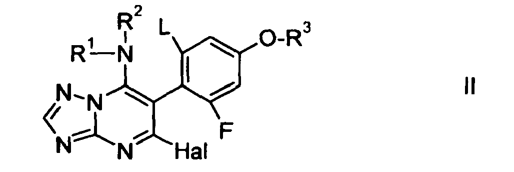 in der Hal für ein Halogenatom steht, mit Verbindungen der Formel III
M-X III
in der M für ein Ammonium-, Tetraalkylammonium- oder Alkali- oder Erdalkalimetall-Kation steht und X die Bedeutung gemäß Anspruch 2 hat.

13. Verfahren zur Herstellung von Verbindungen der Formel I gemäß Anspruch 1, in der X C₁-C₄-Alkyl bedeutet, durch Umsetzung von 2-Aminotriazol der Formel IV 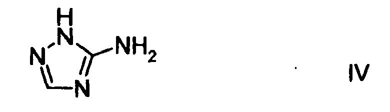 mit Ketoestern der Formel V, 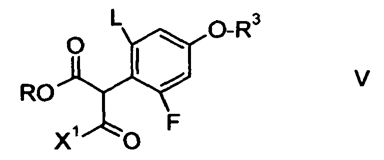 in der R und X¹ unabhängig voneinander C₁-C₄-Alkyl bedeuten, zu 5-Alkyl-7-hydroxy-6-phenyltriazolopyrimidinen der-Formel VI, 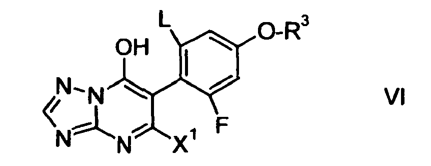 Halogenierung von VI mit Halogenierungsmitteln zu Halogenpyrimidinen der Formel VII, 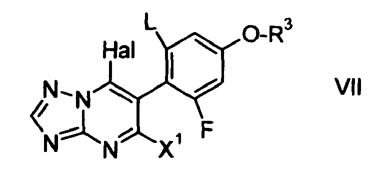 in der Hal für ein Halogenatom steht, und Umsetzung von VII mit Aminen der Formel VIII, 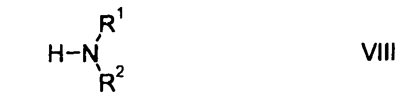 in der R¹ und R² die Bedeutung wie in Formel I haben.

14. Mittel, enthaltend einen festen oder flüssigen Trägerstoff und eine Verbindung der Formel 1 gemäß Anspruch 1 oder 2.

15. Saatgut, enthaltend eine Verbindung der Formel I gemäß Anspruch 1 oder 2 in einer Menge von 1 bis 1000 g/100 kg.

16. Verfahren zur Bekämpfung von pflanzenpathogenen Schadpilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß Anspruch 1 oder 2 behandelt.

## Claims

1. A 6-(2-fluoro-4-alkoxyphenyl)triazolopyrimidine of the formula I 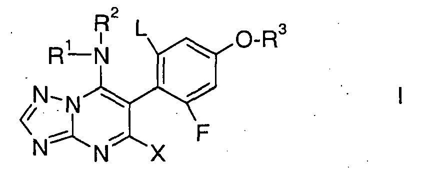 in which the substituents are as defined below:
R¹ C₁-C₈-alkyl, C₁-C₈-haloalkyl, C₃-C₈-cycloalkyl, C₃-C₈-halocycloalkyl, C₂-C₈-alkenyl, C₂-C₈-haloalkenyl, C₃-C₆-cycloalkenyl, C₃-C₆-halocycloalkenyl, C₂-C₈-alkynyl, C₂-C₈-haloalkynyl or phenyl, naphthyl, or a five- or six-membered saturated, partially unsaturated or aromatic heterocycle which contains one to four heteroatoms from the group consisting of O, N and S,
R² is hydrogen or one of the groups mentioned under R¹,
R¹ and R² together with the nitrogen atom to which they are attached may also form a five- or six-membered heterocyclyl or heteroaryl which is attached via N and may contain one to three further heteroatoms from the group consisting of O, N and S as ring member and/or may carry one or more substituents from the group consisting of halogen, C₁-C₆-alkyl, C₁-C₆-haloalkyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, (exo)-C₁-C₆-alkylene and oxy-C₁-C₃-alkyleneoxy;
R³ is C₁-C₈-alkyl, C₁-C₈-haloalkyl, C₃-C₈-alkenyl, C₃-C₈-haloalkenyl, C₃-C₈-alkynyl, C₃-C₈-haloalkynyl, phenyl, phenyl-C₁-C₄-alkyl, mono- or di-(C₁-C₄-alkoxy)-C₁-C₄-alkyl;
R¹, R² and/or R³ may carry one to four identical or different groups R^{a}:
R^{a} is halogen, cyano, nitro, hydroxyl, C₁-C₆-alkyl, C₁-C₆-haloalkyl, C₁-C₆-alkylcarbonyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₈-alkenyl, C₂-C₈-haloalkenyl, C₂-C₆-alkenyloxy, C₂-C₈-alkynyl, C₂-C₈-haloalkynyl, C₃-C₆-alkynyloxy, oxy-C₁-C₃-alkyleneoxy, C₃-C₈-cycloalkenyl, phenyl, naphthyl, a five- or six-membered saturated, partially unsaturated or aromatic heterocycle which contains one to four heteroatoms from the group consisting of O, N and S, where these aliphatic, alicyclic or aromatic groups for their part may be partially or fully halogenated;
L is hydrogen, fluorine or chlorine; and
X is cyano, C₁-C₄-alkyl or C₁-C₄-alkoxy.

2. The compound of the formula I according to claim 1 in which X is cyano or C₁-C₄-alkoxy.

3. The compound of the formula I according to claim 1 or 2 in which X is cyano.

4. The compound of the formula I according to claim 1 in which X is methyl.

5. The compound of the formula I according to claim 1 or 2 in which X is methoxy.

6. The compound of the formula I according to any of claims 1 to 5 in which R¹ and R² are as defined below:
R¹ is CH(CH₃)-CH₂CH₃, CH(CH₃)-CH(CH₃)₂, CH(CH₃)-C(CH₃)₃, CH(CH₃)-CF₃, CH₂C(CH₃)=CH₂, CH₂CH=CH₂, cyclopentyl, cyclohexyl;
R² is hydrogen or methyl; or
R¹ and R² together form -(CH₂)₂CH(CH₃)(CH₂)₂-, -(CH₂)₂CH(CF₃)(CH₂)₂- or -(CH₂)₂O(CH₂)₂-.

7. The compound of the formula I according to claim 1 or 2,
in which
R¹ is a group 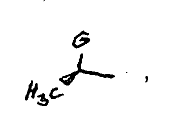
G is C₂-C₆-alkyl, C₁-C₄-alkoxymethyl or C₃-C₆-cycloalkyl;
R² is hydrogen or methyl; and
X is cyano, methyl, methoxy or ethoxy and
L and R³ are as defined in claim 1.

8. The compound of the formula I according to claim 1 or 2,
R¹ is a group 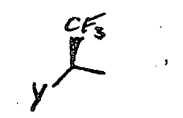
in which Y is hydrogen or C₁-C₄-alkyl and X is cyano, methyl, methoxy or ethoxy and L and R³ are as defined in claim 1.

9. The compound of the formula I according to claim 1 or 2,
in which
R¹ and R² together with the nitrogen and D form a heterocyclic ring where
D together with the nitrogen atom forms a five- or six-membered heterocyclyl or heteroaryl which is attached via N and may contain a further heteroatom from the group consisting of O, N and S as ring member and/or may carry one or more substituents from the group consisting of halogen, C₁-C₄-alkyl, C₁-C₄-alkoxy and C₁-C₂-haloalkyl;
X is cyano, methyl, methoxy or ethoxy and
L and R³ are as defined in claim 1.

10. The compound according to claim 9, in which L is hydrogen and R³ is methyl.

11. The compound of the formula I according to any of claims 1 to 9, in which L is fluorine and R³ is methyl.

12. A process for preparing the compounds of the formula I according to claim 2 which comprises reacting 5-halo-6-(2-halo-4-alkoxyphenyl)triazolopyrimidines of the formula II 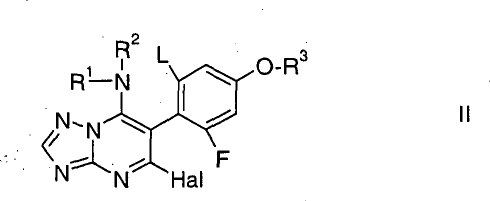 in which Hal is a halogen atom with compounds of the formula III
M-X III
in which M is an ammonium, tetraalkylammonium or alkali metal or alkaline earth metal cation and X is as defined in claim 2.

13. A process for preparing compounds of the formula I according to claim 1 in which X is C₁-C₄-alkyl, by reacting 2-aminotriazole of the formula IV 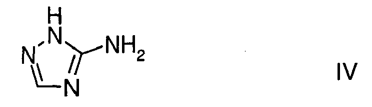 with keto esters of the formula V 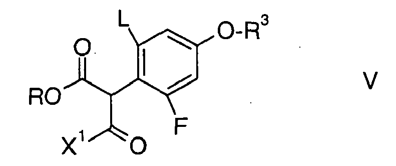 in which R and X¹, independently of one another, are C₁-C₄-alkyl, to give 5-alkyl-7-hydroxy-6-phenyltriazolopyrimidines of the formula VI 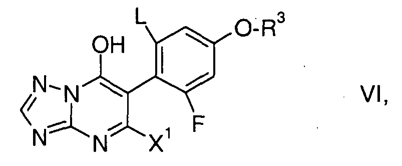 halogenating VI with halogenating agents to give halopyrimidines of the formula VII 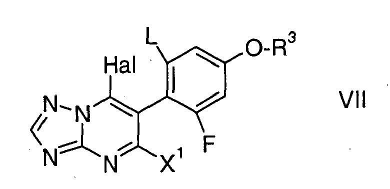 in which Hal is a halogen atom, and reacting VII with amines of the formula VIII 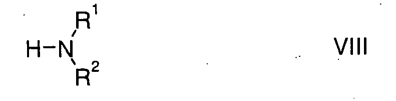 in which R¹ and R² are as defined in formula 1.

14. A composition, comprising a solid or liquid carrier and a compound of the formula I according to claim 1 or 2.

15. Seed, comprising a compound of the formula I according to claim 1 or 2 in an amount of from 1 to 1000 g/100 kg.

16. A method for controlling phytopathogenic harmful fungi, which method comprises treating the fungi or the materials, plants, the soil or seed to be protected against fungal attack with an effective amount of a compound of the formula I according to claim 1 or 2.

## Revendications

1. 6-(2-fluoro-4-alcoxyphényl)triazolo-pyrimidines de formule I : 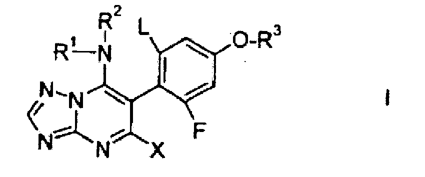 dans laquelle les substituants ont la signification suivante :
R¹ représente un groupement alkyle en C₁-C₈, halogénoalkyle en C₁-C₈, cycloalkyle en C₃-C₈, halogénocycloalkyle en C₃-C₈, alcényle en C₂-C₈, halogénoalcényle en C₂-C₈, cycloalcényle en C₃-C₆, halogénocycloalcényle en C₃-C₆, alcynyle en C₂-C₈, halogénoalcynyle en C₂-C₈ ou phényle, naphtyle ou un hétérocycle à cinq ou six éléments saturé, partiellement insaturé ou aromatique, contenant un à quatre hétéroatomes choisis dans le groupe comprenant O, N ou S,
R² représente l'hydrogène ou l'un des groupements cités pour R¹,
R¹ et R² peuvent également former conjointement avec l'atome d'azote auquel ils sont liés, un système hétérocyclyle ou hétéroaryle à cinq ou
six éléments, qui est relié via N, et qui peut contenir, comme éléments de cycle, un à trois autres hétéroatomes choisis dans le groupe comprenant O, N et S, et/ou qui peut porter un ou plusieurs substituants choisis dans le groupe constitué d'un halogène, d'un groupement alkyle en C₁-C₆, halogénoalkyle en C₁-C₆, alcényle en C₂-C₆, halogénoalcényle en C₂-C₆, alcoxy en C₁-C₆, halogénoalcoxy en C₁-C₆, alcényloxy en C₃-C₆, halogénoalcényloxy en C₃-C₆, (exo) alkylène en C₁-C₆ et oxyalkylène(C₁-C₃)-oxy ;
R³ représente un groupement alkyle en C₁-C₈, halogénoalkyle en C₁-C₈, alcényle en C₃-C₈, halogénoalcényle en C₃-C₈, alcynyle en C₃-C₈, halogénoalcynyle en C₃-C₈, phényle, phénylalkyle en C₁-C₄, mono- ou di-(alcoxy en C₁-C₄)-alkyle(C₁-C₄) ;
R¹, R² et/ou R³ peuvent porter un à quatre groupements R^{a} identiques ou différents :
R^{a} représentant un halogène, un groupement cyano, nitro, hydroxyle, alkyle en C₁-C₆, halogénoalkyle en C₁-C₆, alkylcarbonyle en C₁-C₆, cycloalkyle en C₃-C₆, alcoxy en C₁-C₆, halogénoalcoxy en C₁-C₆, alcoxycarbonyle en C₁-C₆, alkylthio en C₁-C₆, alkylamino en C₁-C₆, dialkylamino en C₁-C₆, alcényle en C₂-C₈, halogénoalcényle en C₂-C₈, alcényloxy en C₂-C₆, alcynyle en C₂-C₈, halogénoalcynyle en C₂-C₈, alcynyloxy en - C₃-C₆, oxyalkylène (C₁-C₃)-oxy, cycloalcényle en C₃-C₈, phényle, naphtyle, hétérocycle à cinq ou six éléments saturé, partiellement insaturé ou aromatique, contenant un à quatre hétéroatomes du groupe comprenant de O, N ou S, les groupements aliphatiques, alicycliques ou aromatiques en question pouvant, eux-mêmes, être partiellement ou complètement halogénés ;
L représente l'hydrogène, le fluor ou le chlore ; et
X représente un groupement cyano, alkyle en C₁-C₄ ou alcoxy en C₁-C₄.

2. Composés de formule I selon la revendication 1, dans laquelle X représente un groupement cyano ou alcoxy en C₁-C₄.

3. Composés de formule I selon la revendication 1 ou 2, dans laquelle X représente un groupement cyano.

4. Composés de formule I selon la revendication 1, dans laquelle X représente un méthyle.

5. Composés de formule I selon la revendication 1 ou 2, dans laquelle X représente un méthoxy.

6. Composés de formule I selon la revendication 1 à 5, dans laquelle R¹ et R² ont la signification suivante :
R¹ représente CH(CH₃)-CH₂CH₃, CH(CH₃)-CH(CH₃)₂, CH(CH₃)-C(CH₃)₃, CH(CH₃)-CF₃, CH₂C(CH₃)=CH₂, CH₂CH=CH₂, un cyclopentyle, un cyclohexyle ;
R² représente l'hydrogène ou un méthyle ; ou
R¹ et R² forment conjointement un radical - (CH₂)₂CH(CH₃)(CH₂)₂-, -(CH₂)₂CH(CF₃)(CH₂)₂- ou - (CH₂)₂O(CH₂)₂-.

7. Composés de formule I selon la revendication 1 ou 2, dans laquelle :
R¹ représente un groupement 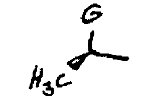
G représente un groupement alkyle en C₂-C₆, alcoxy-méthyle en C₁-C₄ ou cycloalkyle en C₃-C₆ ;
R² représente l'hydrogène ou un méthyle ; et
X représente un groupement cyano, méthyle, méthoxy ou éthoxy, et
L et R³ sont définis selon la revendication 1.

8. Composés de formule I selon la revendication 1 ou 2, dans laquelle :
R¹ représente un groupement 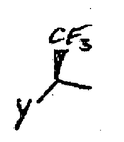 dans lequel Y représente l'hydrogène ou un alkyle en C₁-C₄ et X représente un groupement cyano, méthyle, méthoxy ou éthoxy et L et R³ sont définis selon la revendication 1.

9. Composés de formule 1 selon la revendication 1 ou 2, dans laquelle R¹ et R² forment conjointement avec l'azote et D un hétérocycle, dans lequel :
D forme conjointement avec l'atome d'azote un système hétérocyclyle ou hétéroaryle à cinq à six éléments, qui est relié via N, et qui peut contenir, comme élément de cycle, un autre hétéroatome choisi dans le groupe comprenant O, N et S, et/ou porter un ou plusieurs substituants choisis dans le groupe constitué d'un halogène, d'un groupement alkyle en C₁-C₄, alcoxy en C₁-C₄ et halogénoalkyle en C₁-C₂ ;
X représente un groupement cyano, méthyle, méthoxy ou éthoxy, et
L et R³ sont définis selon la revendication 1.

10. Composés selon la revendication 9, dans lesquels L représente l'hydrogène et R³ représente un méthyle.

11. Composés de formule I selon l'une quelconque des revendications 1 à 9, dans lesquels L est un fluor et R³ représente un méthyle.

12. Procédé de fabrication des composés de formule I selon la revendication 2, en faisant réagir des 5-halogéno-6-(2-halogéno-4-alcoxyphényl)-triazolo-pyrimidines de formule II : 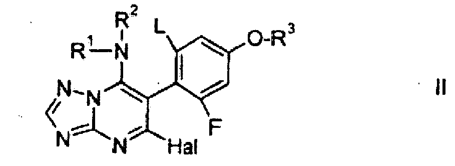 dans laquelle Hal représente un atome d'halogène, avec des composés de formule III :
M-X III
dans laquelle M représente un cation ammonium, un cation tétraalkylammonium ou un cation alcalin ou alcalinoterreux et X a la signification selon la revendication 2.

13. Procédé de fabrication de composés de formule I selon la revendication 1, dans laquelle X représente un alkyle en C₁-C₄, en faisant réagir du 2-aminotriazole de formule IV : 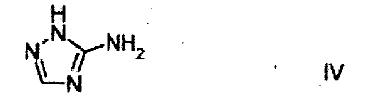 avec des cétoesters de formule V : 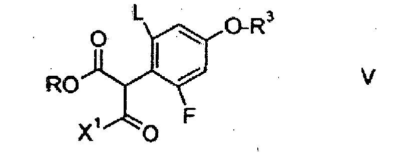 dans laquelle R et X¹ représentent, indépendamment l'un de l'autre, un alkyle en C₁-C₄, pour former les 5-alkyl-7-hydroxy-6-phényltriazolopyrimidines de formule VI : 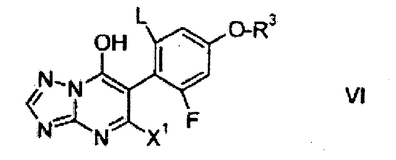 puis en effectuant l'halogénation du composé de formule VI avec des agents d'halogénation pour donner les halogénopyrimidines de formule VII : 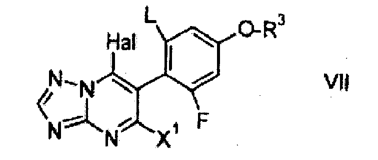 dans laquelle Hal représente un atome d'halogène, et en faisant réagir le composé de formule VII avec des amines de formule VIII : 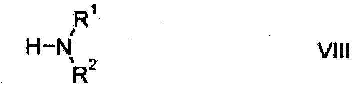 dans laquelle R¹ et R² ont la même signification que dans la formule I.

14. Agent contenant un véhicule solide ou liquide et un composé de formule I selon la revendication 1 ou 2.

15. Semence contenant un composé de formule I selon la revendication 1 ou 2, en quantité de 1 à 1000 g/100 kg.

16. Procédé pour lutter contre des champignons nuisibles phytopathogènes, **caractérisé en ce que** l'on traite les champignons ou les matériaux, les plantes, le sol ou les semences que l'on souhaite protéger d'une attaque fongique, avec une quantité efficace d'un composé de formule I selon la revendication 1 ou 2.
